# EUROPEAN PATENT APPLICATION

(11) **EP 3 650 558 A1**
(43) Date of publication of application: **13.05.2020**
(21) Application number: 18204787.8
(22) Date of filing: 07.11.2018
(51) Int. Cl.: C12Q 1/6827

(54) **LIQUID SAMPLE WORKFLOW FOR NANOPORE SEQUENCING**

(71) Applicant: Siemens Healthcare GmbH, 91052 Erlangen (DE)
(72) Inventor: Huang, Yiwei, 91058 Erlangen (DE); Dietrich, Carsten, 90408 Nürnberg (DE); Mach, Tivadar, 90409 Nürnberg (DE); Prause, Stefan, 91052 Erlangen (DE); Würstle, Maximilian, 91083 Baiersdorf (DE)

(57) **Abstract**

The present invention relates to a method of characterizing a target DNA polynucleotide using rolling circle amplification (RCA) and a synthetic single guide RNA (sgRNA) to identify and cleave the WT version of the target DNA polynucleotide. Also provided are characterization steps based on the use of a transmembrane pore and a DNA translocase enzyme controlling the movement of the DNA polynucleotide through the transmembrane pore. Further envisaged is a kit comprising one or more oligonucleotides specific for at least a portion of the target DNA polynucleotide, an sgRNA specific for the WT version of the target DNA polynucleotide and an sgRNA-guided nucleic acid-binding protein.

## Description

### TECHNICAL FIELD

The present invention relates to a method of characterizing a target DNA polynucleotide using rolling circle amplification (RCA) and a synthetic single guide RNA (sgRNA) to identify and cleave the WT version of the target DNA polynucleotide. Also provided are characterization steps based on the use of a transmembrane pore and a DNA translocase enzyme controlling the movement of the DNA polynucleotide through the transmembrane pore. Further envisaged is a kit comprising one or more oligonucleotides specific for at least a portion of the target DNA polynucleotide, an sgRNA specific for the WT version of the target DNA polynucleotide and an sgRNA-guided nucleic acid-binding protein.

### BACKGROUND

Next-generation sequencing (NGS) is a major driver in genetics and molecular research, including modern diagnostics inter alia in the field of cancer medicine. The technology provides a powerful way to study DNA or RNA samples. New and improved methods and protocols have been developed to support a diverse range of applications, including the analysis of genetic variations and sample specific differences. To improve this approach, methods have been developed that aim at a targeted enrichment of sequencing libraries by focusing on specific sequences, transcripts, genes or genome subregions, or by eliminating undesirable sequences.

Targeted enrichment can be useful in a number of situations where, for example, particular portions of a whole genome need to be analyzed. The efficient sequencing of a complete exome (all transcribed sequences) is a typical example for this approach. Further examples include the enrichment of specific transcripts, the enrichment of mutation hotspots or the exclusion of disturbing nucleic acid species.

Current techniques for targeted enrichment include (i) Hybrid capture, wherein nucleic acid strands derived from the input sample are hybridized specifically to pre-prepared DNA fragments complementary to the targeted regions of interest, either in solution or on a solid support, so that one can physically capture and isolate the sequences of interest; (ii) Selective circularization or molecular inversion probes (MIPs), wherein single-stranded DNA circles that include target region sequences are formed by gap-filling and ligation chemistries in a highly specific manner, creating structures with common DNA elements that are then used for selective amplification of the targeted regions of interest; and (iii) Polymerase Chain Reaction (PCR) amplification, wherein PCR is directed toward the targeted regions of interest by conducting multiple long-range PCRs in parallel, a limited number of standard multiplex PCRs or highly multiplexed PCR methods that amplify very large numbers of short fragments (Mertes et al., 2011, Briefings in functional Genomics, 10, 6, 374-386).

However, in order to make use of these techniques, it is necessary to firstly obtain suitable biopsy material from a patient, in particular if the approaches are used in cancer diagnostics. Solid tissue biopsies are costly and in many cases painful for the patient. Moreover, solid tissue biopsies cannot always be performed because they cannot reflect current disease dynamics or sensitivity to treatment, e.g. in the case of cancer. It is hence necessary to provide an alternative to the solid tissue biopsies and, at the same time, to increase the sensitivity of the method. One emerging solution to combat both the sensitivity limitations of NGS and the invasiveness of acquiring tissue samples is enriching liquid biopsies (Hesse et al., 2015, Advances in Molecular Diagnostics, 1, 1, 2-7). Liquid biopsies are typically blood samples from which either circulating cell, e.g. circulating tumor cells (CTC), or circulating cell-free DNA (cfDNA) can be isolated. These cell-free DNAs (cfDNA) or circulating nucleic acids (including DNA, as well as RNA species such as micoRNA) remain as circulating fragments in the blood for some time and, like other blood analytes, can be assessed by simple blood sampling. Yet, cfDNA and similar circulating nucleic acids are a challenging analyte since they are very variable in plasma and vary not only from person to person, but also depending on the disease status. For example, cfDNA levels in plasma are usually limited to 1 to 100 ng / ml plasma and, in addition, the signal-to-noise ratio between cfDNA fragments and normal cfDNA is low. cfDNA and other circulating nucleic acids fragments are also quite small with a mean size of about 60-180 bp and require specific extraction and NGS library size selection protocols.

There is hence a need for a streamlined, cost- and resource-sensitive enrichment and sequencing approach, which allows for an efficient characterization of target DNA polynucleotides, in particular target DNA polynucleotides derived from liquid biopsies.

### SUMMARY

The present invention addresses this need and provides a method of characterizing a target DNA polynucleotide comprising (i) providing a mixture of DNA polynucleotides comprising at least a wildtype (WT) version and a mutant version of said DNA polynucleotide; (ii) providing a pool of amplified and concatenated DNA polynucleotides by amplifying said mixture of DNA polynucleotides of step (i) by rolling circle amplification (RCA); (iii) identifying and cleaving the WT version of the target DNA polynucleotide by using a synthetic single guide RNA (sgRNA) specific for said WT version and an sgRNA-guided nucleic acid-binding protein, preferably Cas9; (iv) size selecting uncut mutant target DNA polynucleotides; and (v) characterizing the uncut mutant target DNA polynucleotides. The method advantageously allows to reduce the sequencing depths due to the removal of WT sequences. The approach is further amenable to multiplexing different patient samples and allows for an enrichment of selected regions or panels of genes or exons.

In a preferred embodiment said step (v) as mentioned above comprises the following sub-steps: (v-a) ligating an adaptor polynucleotide associated with an DNA translocase enzyme and at least one cholesterol tether segment to the mutant target DNA polynucleotides obtained in step (iv); (v-b) contacting the modified DNA polynucleotide obtained in step (v-a) with a transmembrane pore such that the DNA translocase controls the movement of the DNA polynucleotide through the transmembrane pore and the cholesterol tether anchors the DNA polynucleotide in the vicinity of the transmembrane pore; and (v-c) taking one or more measurements during the movement of the DNA polynucleotide through said transmembrane pore, wherein the measurements are indicative of one or more characteristics of the DNA polynucleotide, thereby characterizing the target DNA polynucleotide. Accordingly, long reads with repeated sequences as obtained with the above described method significantly improve sequencing accuracy for mutation calling in the transmembrane pore based sequencing.

In a further preferred embodiment, the methods as mentioned above additionally comprises after step (i) a step (i-a) of end-repairing and A-tailing of the DNA polynucleotide.

In yet another preferred embodiment, the methods as mentioned above, additionally comprise after step (i-a) a step (i-b) of circularizing the DNA polynucleotide with a stem-loop oligonucleotide, wherein said stem-loop oligonucleotide comprises a barcoding sequence and a restriction enzyme recognition site.

It is particularly preferred that the rolling circle amplification is performed with one or more oligonucleotides specific for at least a portion of the target DNA polynucleotide. In a further specific embodiment of the present invention said one or more oligonucleotides specific for at least a portion of the target DNA polynucleotide are hexamers, heptamers, and/or octamers.

In another embodiment of the present invention the rolling circle amplification is performed until the amplified DNA polynucleotide has a size of at least about 300 nucleotides. It is particularly preferred that it has a size of about at least 3000 nucleotides.

In yet another embodiment the rolling circle amplification products obtained are repaired using a T7 endonuclease, DNA polymerase and optionally a ligase.

In another embodiment said target DNA polynucleotide represents a gene, one or more exons of a gene, an intergenic region, a non-transcribed regulatory region, and/or an open reading frame or a sub-portion thereof; or a panel of different genes, a panel of one or more exons of different genes, a panel of intergenic regions, a panel of non-transcribed regulatory regions, and/or a panel of open reading frames or sub-portions thereof, or any combination of any of the before mentioned elements.

It is preferred that the said target DNA polynucleotide is cell free DNA (cfDNA). It is particularly preferred that said cfDNA is derived from a liquid biopsy.

In a specific embodiment of the methods of the present invention said characterization of the DNA polynucleotide is (i) a determination of the length of the DNA polynucleotide, (ii) a determination of the identity of the DNA polynucleotide, or (iii) a determination of the sequence of the DNA polynucleotide. It is particularly preferred that the sequence of the DNA polynucleotide is determined.

In a specific embodiment of the method making use of a transmembrane pore as defined above, the DNA translocase is a DNA helicase such as Hel308 helicase, RecD helicase, XPD helicase or Dda helicase.

In yet another embodiment of said method said transmembrane pore is a protein pore derived from hemolysin, leukocidin, MspA, MspB, MspC, MspD, CsgG, lysenin, outer membrane porin F (OmpF), outer membrane porin G (OmpG), outer membrane phospholipase A, Neisseria autotransporter lipoprotein (NalP) or WZA.

In another aspect the present invention relates to a kit for characterizing a target DNA polynucleotide comprising one or more oligonucleotides specific for at least a portion of the target DNA polynucleotide, a synthetic single guide RNA (sgRNA) specific for the WT version of the target DNA polynucleotide and an sgRNA-guided nucleic acid-binding protein. It is particularly preferred that the sgRNA-guided nucleic acid-binding protein is a Cas9 endonuclease.

In a specific embodiment of said kit the kit additionally comprises a DNA translocase and a cholesterol tether.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

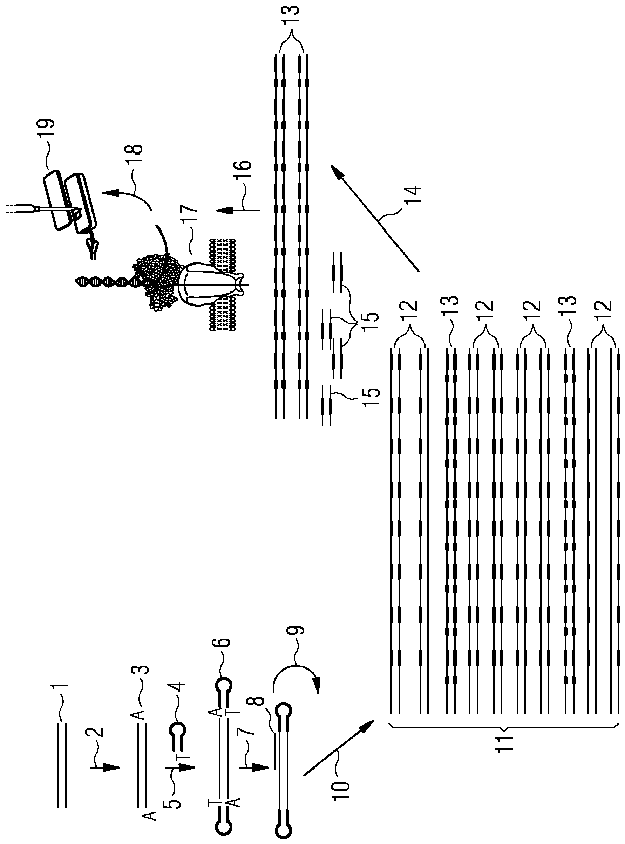

FIG. 1 shows a schematic illustration of the steps for characterizing a target DNA polynucleotide using rolling circle amplification (RCA) and a synthetic single guide RNA (sgRNA) according to an embodiment of the present invention.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", " (iii) " or "(a)", "(b)", "(c)", "(d)", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only, and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

As has been set out above, the present invention concerns in one aspect a method of characterizing a target DNA polynucleotide comprising (i) providing a mixture of DNA polynucleotides comprising at least a wildtype (WT) version and a mutant version of said DNA polynucleotide; (ii) providing a pool of amplified and concatenated DNA polynucleotides by amplifying said mixture of DNA polynucleotides of step (i) by rolling circle amplification (RCA); (iii) identifying and cleaving the WT version of the target DNA polynucleotide by using a synthetic single guide RNA (sgRNA) specific for said WT version and an sgRNA-guided nucleic acid-binding protein, preferably Cas9; (iv) size selecting uncut mutant target DNA polynucleotides; and (v) characterizing the uncut mutant target DNA polynucleotides, preferably by sequencing.

The term "target DNA polynucleotide" as used herein relates to any DNA molecule of interest, which is amenable to molecular analysis. In specific embodiments of the present invention the target polynucleotide represents a gene, one or more exons of a gene, an intergenic region, a non-transcribed regulatory region, and/or an open reading frame or a sub-portion thereof. In further embodiments, the target polynucleotide may also be a panel of different genes, a panel of one or more exons of different genes, a panel of intergenic regions, a panel of non-transcribed regulatory regions, and/or a panel of open reading frames or sub-portions thereof. The target DNA polynucleotide may further be provided as single DNA molecule or is provided, preferably, in the form of a pool of DNA molecules, e.g. representing a gene, one or more exons of a gene, an intergenic region, a non-transcribed regulatory region, and/or an open reading frame or a sub-portion thereof as mentioned above, or a panel of different genes, a panel of one or more exons of different genes, a panel of intergenic regions, a panel of non-transcribed regulatory regions, and/or a panel of open reading frames or sub-portions thereof.

In a first step of the method of the present invention a mixture of DNA polynucleotides comprising at least a wildtype (WT) version and a mutant version of said DNA polynucleotide is provided.

The "DNA polynucleotide" may be naturally occurring or be artificial. It may comprise modifications such as oxidized or methylated nucleotides. The DNA polynucleotide may also, in certain embodiments, comprise artificial additions such as tags or labels.

The DNA polynucleotide may be of any possible origin, e.g. prokaryotic, eukaryotic, archaeal or viral. The DNA polynucleotide to be characterized according to the present invention may have any known possible biological or cellular function. For example it may be any naturally occurring or synthetic polynucleotide.

The provision of the DNA polynucleotide may include the extraction and/or purification of the DNA molecule, separation from cell debris, filtration, elution from a column, e.g. silica membrane columns, centrifugation, digestion, e.g. RNase digestion, or removal of nucleotide or protein components in a sample etc. It is preferred that the DNA polynucleotide is provided in a buffer solution comprising any suitable ingredient preventing DNA degradation. The buffer may, for example, be a H₂O buffer comprising EDTA (e.g. 0.1 mM) or a TE buffer (10mM Tris, 1mM EDTA). The buffer may preferably comprise DNAse blocking compounds or DNase inhibitors. Also envisaged is the provision of DNA polynucleotides obtained from RNA polynucleotides, e.g. via reverse transcription.

The provision of DNA polynucleotides may also involve the taking of samples from a subject and their processing, e.g. extraction of DNA or preparatory steps facilitating the extraction of DNA. The term "sample from a subject" as used herein relates to any biological material obtained via suitable methods known to the person skilled in the art from a subject. The sample used in the context of the present invention should preferably be collected in a clinically acceptable manner, more preferably in a way that DNA polynucleotides are preserved. The biological samples may include body tissues and/or fluids, such as blood, or blood components like serum or plasma, sweat, sputum or saliva, semen and urine, as well as feces or stool samples. It is particularly preferred that the sample is a liquid biopsy sample.

The term "liquid biopsy" as used herein relates to sampling and analysis of non-solid biological tissue, primarily blood. The sampling is largely non-invasive which allows to repeat it frequently and thus helps to track mutations or modifications over time, or to validate efficiency of treatments. The liquid biopsy sampling typically aims at obtaining different species of cells and/or nucleic acids. For example, circulating endothelia cells (CECs) or cell-free fetal DNA (cffDNA) may be sampled. It is preferred that circulating tumor cells (CTC) and, in particular, cell free DNA be sampled. Accordingly, in a particularly preferred embodiment, the DNA polynucleotide to be analyzed according to the present invention is cell free DNA.

In further embodiments the biological sample may contain a cell extract derived from or a cell population including an epithelial cell, preferably a neoplastic epithelial cell or an epithelial cell derived from tissue suspected to be neoplastic. Alternatively, the biological sample may be derived from the environment, e.g. from the soil, a lake, a river etc., or from animal sources.

In certain embodiments cells may be used as primary sources for DNA polynucleotides. Accordingly the cells may be purified from obtained body tissues and fluids if necessary, and then further processed to obtain DNA polynucleotides. In certain embodiments samples, in particular after initial processing, may be pooled. The present invention preferably envisages the use of non-pooled samples.

In a specific embodiment of the present invention the content of a biological sample may also be submitted to an enrichment step. For instance, a sample may be contacted with ligands specific for the cell membrane or organelles of certain cell types, functionalized for example with magnetic particles. The material concentrated by the magnetic particles may subsequently be used for the extraction of DNA polynucleotides. In further embodiments of the invention, biopsy or resections samples may be obtained and/or used. Such samples may comprise cells or cell lysates. Furthermore, cells, e.g. tumor cells, may be enriched via filtration processes of fluid or liquid samples, e.g. blood, urine, sweat etc. Such filtration processes may also be combined with enrichment steps based on ligand specific interactions as described herein above.

A "mixture" of DNA polynucleotides as used herein refers to a situation in which a sample or any starting composition comprises at least two species of a target DNA polynucleotide, a wildtype (WT) and a mutant version. The term "wildtype version of a target DNA polynucleotide" as used herein relates to the typical form of DNA polynucleotide, e.g. gene, exon, open reading frame etc. as it occurs typically in nature, e.g. in a healthy individual if the DNA polynucleotide is associated with a disease or in a majority of individuals of a population of individuals. A "mutant version of a target DNA polynucleotide" is accordingly a version, which has undergone a change in its molecular structure, e.g. sequence, in comparison to the WT version. For example, in case of a DNA polynucleotide associated with a disease the mutant version of the DNA polynucleotide may be associated with the occurrence of the disease, whereas the WT version may be associated with a healthy state. Apart from said difference both molecules are typically identical or at least highly similar. Within the context of the present invention both are hence considered to be target DNA polynucleotides. The mixture of both entities as mentioned above may have any proportion allowing for an identification of both entities.

In a specific embodiment, after the provision of a mixture of DNA polynucleotides comprising at least a WT version and a mutant version of the target DNA polynucleotide an optional step of end-repairing and A-tailing of said DNA polynucleotide is performed. This step intends to convert DNA polynucleotides with blunt, or protruding 3' or 5' ends into DNA polynucleotides comprising 3' A overhang which is phosphorylated and can be used for subsequent ligation reactions. The performance of this step largely depends on the form and origin of the DNA polynucleotides; it may, in certain embodiments, also be modified and/or adapted to necessities. For example, if there is no need for an end repairing step or there are already suitable blunt ends present on the DNA polynucleotide, the end-repairing activity may not be used. Similarly, in case there is already a suitable A overhang in the DNA polynucleotide, there is no need for an A-tailing activity which can accordingly be skipped. The end-repairing may be performed by with any suitable end-repairing enzymatic activity, e.g. DNA polymerase I, preferably the Klenow fragment thereof, T4 DNA polymerase or T4 polynucleotide kinase. It is preferred that the end repairing is performed with T4 DNA polymerase, T4 PNK and Klenow at 20 °C. The A-tailing activity may be performed by any suitable A-tailing enzymatic activity such as Taq DNA polymerase or Klenow fragment. The A-tailing is preferably carried out with Taq DNA polymerase at 65°C. Further details can be derived from suitable literature sources such as Nucleic Acids Research, 2010, 38, 13, e137.

In a further preferred embodiment after the step of end-repairing and A-tailing of said DNA polynucleotide, or if the DNA polynucleotide already comprises a suitable A overhang, without said step of end-repairing and A-tailing, a step of circularizing the DNA polynucleotide with a stem-loop oligonucleotide is performed.

Typically, a stem-loop oligonucleotide is first connected to said DNA polynucleotides. The connection preferably takes place at both termini of the DNA polynucleotide. It is further preferred that the connection makes use of a 3' overhang nucleotide at the 3' termini of the double stranded DNA polynucleotide, more preferably at the 3' A overhang of the double stranded DNA polynucleotide. In a typical embodiment, the stem-loop oligonucleotide comprises a 3' overhang which is compatible to the corresponding overhang at the DNA polynucleotide. In case of a 3' A overhang at the DNA polynucleotide the stem-loop oligonucleotide may comprise a complementary 3' T overhang. The term "connection" as used herein relates to an annealing reaction of the stem-loop oligonucleotide followed by a suitable bond forming reaction, typically a ligation, of the annealed stem-loop oligonucleotide. The ligation may be a chemical or an enzymatic ligation. The enzymatic ligation is preferred. A chemical ligation typically requires the presence of condensing reagents. An example of a chemical ligation envisaged by the present invention makes use of electrophilic phosphorothioester groups. Further examples include the use of cyanogen bromide as a condensing agent. The enzymatic ligation may be performed with any suitable enzymatic ligase known to the skilled person. Examples of suitable ligases include T4 DNA ligase, E. coli DNA ligase, T3 DNA ligase and T7 DNA ligase. Alternatively, ligases such as Taq DNA ligase, Tma DNA ligase, 9°N DNA ligase, T4 Polymerase 1, T4 Polymerase 2, or Thermostable 5' App DNA/RNA ligase may be used.

In another step, which may be performed after the connection with the stem-loop oligonucleotide, or alternatively without said connection, i.e. with the DNA polynucleotide not connected to the stem-loop oligonucleotide, the DNA polynucleotide is circularized. The term "circularization" as used herein relates to the conversion of a linear nucleic acid molecule to a circular nucleic acid molecule. The circularization may, in principle, be achieved by connecting both termini of the polynucleotide, or by melting said polynucleotide while keeping the coherence at the 3' and 5' termini via the presence of a loop element. It is preferred that the loop element based strategy is followed. The circulation, in this embodiment, involves a melting step, e.g. an increase of the reaction temperature to the melting temperate of the DNA polynucleotide. The resulting molecule is a circular ssDNA polynucleotide.

It is particularly preferred that the strand separation and conversion into a circular ssDNA molecule is assisted by the previous connection of the DNA polynucleotide and the stem-loop oligonucleotide as defined herein.

The term "stem-loop oligonucleotide" as used herein refers to a nucleic acid, typically a DNA oligonucleotide, comprising a partially double-stranded segment which comprises a double stranded stem sector and a hairpin or hairpin loop sector connecting the double stranded sectors. The stem part thus typically comprises two regions of the same strand, which are complementary in nucleotide sequence when read in opposite directions. These segments can base-pair and form a double helix that ends in an unpaired loop.

Without wishing to be bound by theory, it is believed that the formation of a stem-loop structure is dependent on the stability of the resulting helix and loop regions. The first prerequisite is typically the presence of a sequence that can fold back on itself to form a paired double helix. The stability of this helix may predominantly be determined by its length, the number of mismatches or bulges it may contain and the base composition of the paired region. Since pairings between guanine and cytosine have three hydrogen bonds they are more stable in comparison to adenine-thymine pairings, which have only two. In certain embodiment, the stem segment comprises more guanine-cytosine pairings than adenine-thymine pairings.

Furthermore, the stability of the loop may have an influence on the formation of the stem-loop structure. It is preferred that the hairpin loop is not smaller than three bases, e.g. are 4, 5, 6, 7, 8 or more bases long. It is further preferred that the loops are not longer than about 10 to 12 bases since large loops typically tend to be unstable. In certain embodiments, the loop may have a size of more than 12 bases and showing a further secondary structure such as a pseudoknot. It is particularly preferred that the loop has a length of about 4-8 bases. In some embodiments, the loop has the sequence 5'-TNCG-3', i.e. is tetraloop which is stabilized due to the base-stacking interactions of its component nucleotides.

The stem-loop oligonucleotide according to the present invention as described above may, in specific embodiments, additionally comprise a barcoding sequence or section. The term "barcoding sequence" or "barcoding section" as used herein relates to a sequence which is artificially included in the polynucleotide and which serves for identification purposes after the characterization step, e.g. after sequencing. The barcoding segment may, thus, inform the user which of several samples is being characterized, e.g. sequenced. A barcoding section accordingly comprises a unique sequence which is provided only once, i.e. for one molecule/polynucleotide as described above only. The barcoding sequence is preferably different from known naturally occurring sequence motifs. In other embodiments, it is preferably long enough to avoid mix-ups with naturally occurring sequences or different barcoding sequences. According to preferred embodiments, the barcoding sequence has a length of at least 6 to about 12 or more nucleotides. In certain embodiments a barcoding segment may be present once, or multiple times in the polynucleotide of the present invention. If more than one barcoding segment is present, e.g. 2, 3, 4 or 5 or more, the differentiating, i.e. indexing sequence of each segment is different, thus allowing for two or more independent identification processes. The barcoding sequence may, for example, advantageously be used to multiplex different patients or different patient samples etc. Further details would be known to the skilled person, or can be derived from suitable literature sources such as Kozarewa et al., 2011, Methods Mol. Biol.733, 279-298.

The stem-loop oligonucleotide according to the present invention as described above may, in specific embodiments, alternatively or additionally comprise a restriction enzyme recognition site. The restriction enzyme recognition site may be located at any suitable position within the stem-loop segment. The restriction enzyme recognition site is preferably located in the stem sector of the stem-loop oligonucleotide. It allows for a cleavage in said oligonucleotide or any molecule connected to it or including it. For example, after having performed RCA as described herein, each repetitive unit of the amplified DNA polynucleotide comprises at least one unit of the restriction enzyme recognition site. It may subsequently be cleaved or cut at any suitable point in time, e.g. if a long concatemer shall be size reduced to shorter fragments or single repetitive units. The term "cleaving" or "cleavage" as used herein refers to a double-stranded cut, i.e. an incision trough each strand, in a double stranded nucleic acid molecule, typically performed by a restriction enzyme or restriction endonuclease. The restriction enzyme to be used for this activity may be any suitable restriction enzyme know to the skilled person. By cutting at the restriction enzyme recognition site any suitable ending at the cleaved site may be produced. Such an ending may either be a sticky ending, i.e. comprising a 5' or 3' overhang, or it may be a blunt end, i.e. having no overhand. It is preferred that a sticky ending is obtained. It is further preferred that the sticky end is a 3' overhang. In particularly preferred embodiments, the overhang is 1 nucleotide 3' overhang. Even more preferred is that the 3' overhang is a 1 nucleotide A overhang. It is accordingly envisaged that the restriction enzyme recognition site is one which, when cleaved by the cognate restriction enzyme, provides a 3' A overhang.

In a specific group of embodiments, the restriction enzyme recognition site may have the sequence 5'-ACAGT-3' or 5'-TCAGA-3'. According to further embodiments, the restriction enzyme recognition site 5'-ACAGT-3' may be cleaved at the third position to yield 5'-ACA / GT-3', thus providing a 1 nucleotide 3' overhang, more specifically to provide a 1 nucleotide 3' A overhang. Enzyme Bst4CI, HpyCH4III and TaaI are known to recognize the restriction enzyme recognition site 5'-ACAGT-3'. These enzymes may thus, preferably, be used within the context of the present invention. According to different embodiments, the restriction enzyme recognition site 5'-TCAGA -3' may be cleaved at the third positon to yield 5'-TCA / GA-3', thus providing a 1 nucleotide 3' overhang, more specifically to provide a 1 nucleotide 3' A overhang. Enzyme Hpy188I is known to recognize the restriction enzyme recognition site 5'-TCAGA-3'. These enzymes may thus, preferably, be used within the context of the present invention.

In a subsequent step of the method of the present invention the DNA polynucleotide is amplified by rolling circle amplification (RCA). The term "rolling circle amplification" or "RCA" as used herein relates to an isothermal enzymatic process where a DNA polynucleotide, which is typically short, is amplified to form a long single stranded DNA polynucleotide using a circular DNA template and a suitable polymerase. The RCA product is typically a concatemer containing several, e.g. 5 to 500 tandem repeats that are complementary to the circular template. Typically, suitable DNA polymerases are used for the process. Examples include Phi29 polymerase, Bst polymerase or exo-DNA polymerase. It is preferred to use Phi29 polymerase. The template for RCA as used in the context of the present invention is a single stranded circular DNA molecule. The reaction is in essence the continuous addition of nucleotides to a primer annealed to said circular ssRNA template. Accordingly, the present invention envisages the conversion of the double stranded DNA polynucleotides obtained in step (i) into circular templates and the conversion of said templates into a concatemeric form, e.g. via the use of one or more suitably annealed oligonucleotide(s).

In a preferred embodiment the RCA is performed with one or more oligonucleotides specific for at least a portion of the target DNA polynucleotide. The term "specific for a target DNA polynucleotide" as used herein relates to a sequence complementarity between the oligonucleotide and the DNA polynucleotide, which allows to anneal said oligonucleotide to the DNA polynucleotide and to subsequently perform an amplification reaction. The term "complementary" or "complementarity" thus refers to the presence of matching base pairs in opposite nucleic acid strands, i.e. in the oligonucleotide and the DNA polynucleotide. For example, to a nucleotide or base A in a sense strand a complementary or antisense strand binds with a nucleotide or base T, or vice versa; likewise to a nucleotide or base G in a sense strand the complementary or antisense strand binds with a nucleotide or base C, or vice versa. This scheme of complete or perfect complementarity may, in certain embodiments of the invention, be modified by the possibility of the presence of single or multiple non-complementary bases or stretches of nucleotides within the sense and/or antisense strand(s). Thus, to fall within the notion of a pair of sense and antisense strands, both strands may be completely complementary or may be only partially complementary, e.g. show a complementarity of about 80%, 85%, 90%, 91%, 92%, 93%, 94%, 95%, 96%, 97%, 98%, 99%, 99.5%, or 100% between all nucleotides of both strands or between all nucleotides in specific segments as defined herein. Non-complementary bases may comprise one of the nucleotides A, T, G, C, i.e. show a mismatch e.g. between A and G, or T and C, or may comprise any modified nucleoside bases including, for example, modified bases as described in WIPO Standard ST.25. Furthermore, the present invention also envisages complementarity between non-identical nucleic acid molecules, e.g. between a DNA strand and a RNA strand, a DNA strand and a PNA strand, a DNA strand and a CNA strand, etc. It is preferred that the complementarity between strands or segments as defined herein is a complete or 100% complementarity.

A "specific" annealing of the oligonucleotide and the DNA polynucleotide means that a complete or partial complementarity/partial matching is possible which allows to recognize the DNA polynucleotide, but which, in certain embodiments, also accepts the presence of non-matching nucleotides. For example, the specific annealing may be possible with WT target DNA polynucleotides as well as with mutant target DNA polynucleotides as defined herein in case the annealing takes place at the differing section of the DNA polynucleotide. Such an annealing is, in particular, envisaged if the mutant differs by single nucleotide polymorphisms, or 2-3 nucleotide divergences. In other embodiments, the specific annealing may involve a complete complementarity which may be implemented by a binding in section of the DNA polynucleotide which is not affected by a sequence modification reflected by the difference of the WT and the mutant version of the target DNA polynucleotide of the present invention.

The term "specific for at least a portion of the target DNA polynucleotide" as used herein means that the oligonucleotide may have, in certain embodiments, at least a complementary overlap with said target DNA polynucleotide. The overlap may, for example, be an overlap of 4, 5, 7, 6, 7, 8, 9, 10, 12, 15, 18, or 20 nucleotides, or any value in between the mentioned values. The overlap may depend on the size of the oligonucleotide and may accordingly be adjusted. Within said overlap the matching or complementarity between the complementary bases is preferably 100%. In alternative embodiments, the matching is less than 100%, e.g. 99%, 95%, 90%, 85% or less than 85%. The specificity of the annealing may further be adjusted via the setting of annealing temperatures, with higher temperatures increasing the specificity. Hybridizing temperatures may be calculated by the skilled person according to known rules largely depending of on the sequences involved. It is particularly preferred that the hybridization conditions and the oligonucleotide design including its length be adapted to the working conditions of polymerases used for RCA as defined herein. For example, in embodiments in which Phi29 polymerase is used, a processing temperature of about 30°C may be used.

In certain alternative embodiments, the oligonucleotide may be specific for the stem-loop oligonucleotide sequence as mentioned above, or it may at least partially bind to at least a portion of said stem-loop oligonucleotide sequence.

In a very specific embodiment, the following steps are performed for RCA: after ligation, the oligonucleotides are added to the ligated DNA. Subsequently a melting step is carried out as described above. After melting, the temperature is decreased and the oligonucleotides are allowed to bind to their target sequence. Subsequently a polymerase, e.g. the Phi29 polymerase, is added in order to amplify the circular template.

In certain embodiments, only one oligonucleotide binding to the target DNA polynucleotide may be used for RCA. In other embodiments, more than one oligonucleotide may be used, e.g. 2, 3, 4, 5 etc. These oligonucleotides may preferably bind at different positons in the target DNA polynucleotide, preferably those which are not affected by a sequence modification reflected by the difference of the WT and the mutant version of the target DNA polynucleotide.

The oligonucleotides may have any suitable size, e.g. from 6 to 30 nucleotides. It is preferred that the oligonucleotide is a hexamer, heptamer or octamer. The use of similar or identical sizes is preferred if more than one different oligonucleotide is used for RCA.

The RCA may be performed at any suitable temperature, e.g. at room temperature or a temperature up to 37°C. The temperature may be a constant temperature. The RCA may further be performed in any suitable environment, e.g. in solution or on a solid support. In specific embodiments, also RCA reactions in complex biological environments such as on a cell surface are envisaged.

In another embodiment the RCA may be performed until the amplified DNA polynucleotide has a certain, preferably predefined, size. The size may be dependent on a subsequent activity planned for the obtained DNA polynucleotide. For example, if the characterization of the DNA polynucleotide is to be performed with transmembrane pore based sequencing technologies, a long amplificate is preferred. If, in alternative embodiments, a different NGS approach is to be performed, which typically requires short input polynucleotides, a short amplificate may be obtained. The size of the amplificate may be at least 300 nucleotides, preferably it may be in a range of about 300 nucleotides to about 10 000 nucleotides, more preferably, it may be in a range of about 300 to 7000 nucleotides, e.g. 300, 400, 500, 600, 700, 800, 900, 1000. 1500, 2000, 2500, 3000, 3500, 4000, 4500, 5000, 5500, 6000, 6500 or 7000 or any value in between the mentioned sizes. In the most preferred embodiment, it may have a size of about at least 3000 nucleotides. In further preferred embodiments, short fragments may also be obtained by cutting longer amplificates, e.g. via restriction enzyme recognition sites present in the stem-loop structure as described herein.

The size of the amplificate may be controlled by any suitable means, e.g. the use of temperature controls, e.g. a heating denaturation step, or the addition of inhibiting molecules, the addition of EDTA, or the addition of proteinases etc. It is preferred to control the size of the amplificate by using a heating denaturation step.

The RCA as envisaged by the present invention may, in certain additional embodiments, also include a multiple amplification step, wherein multiple oligonucleotides, e.g. as defined herein, hybridize or anneal with the same target DNA polynucleotide circle, thus allowing for the production of multiple RCA products at the same time. Similarly, a hyperbranched RCA may be performed where the RCA product is used as template for further amplification with a second or third set of oligonucleotides.

Also envisaged in the present invention is the monitoring and detection of the RCA process. This may be implemented by incorporating fluorescent dyes to the RCA product, e.g. via fluorophore-conjugated dTNPs or the hybridization with fluorophore-tethered complementary strands. The detection may accordingly be performed with the help of fluorescence spectroscopy, flow cytometry or microscopy. Also the employment of gold nanoparticles, magnetic beads or quantum dots is envisaged for the detection of RCA products. Preferably, the monitoring and detection of the RCA process is performed with the help of gel electrophoresis analyses. Further RCA details may be derived from suitable literature sources such as Ali et al., 2014, Chem. Soc. Rev., 43, 3324-3341.

In a specific embodiment, the rolling circle amplification products obtained may be repaired using a T7 endonuclease and DNA polymerase. For example, T7 endonuclease I and T4 DNA polymerase activities may be used to remove mismatch structures and for repairing purposes. Optionally, also a ligase activity may be used for this purpose.

In a further step of the method of the present invention a WT version of the target DNA polynucleotide is identified and cleaved using a synthetic single guide RNA (sgRNA) specific for said WT version and an sgRNA-guided nucleic acid-binding protein.

This step is, in general, based on the employment of the CRISPR/Cas system. The term "CRISPR/Cas system" as used herein relates to a biochemical method to specifically cut and modify nucleic acids. For example, genes in a genome can generally be inserted, removed or switched off with the CRISPR/Cas system, nucleotides in a gene or nucleic acid molecule can also be changed. The effect of the concept and activity steps of the CRISPR/Cas system has various similarities to that of RNA interference, since short RNA fragments of about 18 to 20 nucleotides mediate the binding to the target in both bacterial defense mechanisms. In the CRSIPR/Cas system typically RNA-guided nucleic acid-binding proteins, such as Cas proteins, bind certain RNA sequences as ribonucleoproteins. For example, a Cas endonuclease (e.g. Cas9, Cas5, Csn1 or Csx12, or derivatives thereof) can bind to certain RNA sequences termed crRNA repeats and cut DNA in the immediate vicinity of these sequences. Without wishing to be bound by theory, it is believed that the crRNA repeat sequence forms a secondary RNA structure and is then bound by the nucleic acid-binding protein (e.g. Cas) which alters its protein folding allowing the target DNA to be bound by the RNA. Furthermore, the presence of a PAM motif, i.e. a protospacer adjacent motif, in the target DNA is necessary to activate the nucleic acid-binding protein (e.g. Cas). The DNA is typically cut three nucleotides before the PAM motif. The crRNA repeat sequence is typically followed by a sequence binding to the target DNA, i.e. a crRNA spacer; both sequences, i.e. the crRNA repeat motif and the target binding segment are usually labelled as "crRNA". This second part of the crRNA (target binding segment) is a crRNA-spacer sequence having the function of a variable adapter. It is complementary to the target DNA and binds to said target DNA. An additional RNA, a tracrRNA, or trans-acting CRISPR RNA, is also required. tracrRNA is partially complementary to crRNA, so that they bind to each other. tracrRNA typically binds to a precursor crRNA, forms an RNA double helix and is converted into the active form by RNase III. These properties allow for a binding to the DNA and a cutting via the endonuclease function of the nucleic acid-binding protein (e.g. Cas) near the binding site.

In this context the term "synthetic single guide RNA (sgRNA)" or "single guide RNA (sgRNA)" as used herein relates to an artificial or synthetic combination of a crRNA and a tracrRNA sequence of the CRISPR/Cas system as described above. Typically, the sgRNA comprises a target specific sequence which can be used to guide a DNA binding protein towards the binding site. This target specific sequence may have any suitable length. It is preferred that said length is between about 19 to 30 nucleotides. More preferably, the sequence has a length of 20 nucleotides.

As described in Jinek et al., 2012, Science, 337, 816- 821 crRNA and tracrRNA can be combined into a functional species (sgRNA) which fulfills both activities (crRNA and tracrRNA) as mentioned above. For example, nucleotides 1-42 of crRNA-sp2, nucleotides 1-36 of crRNA-sp2 or nucleotides 1-32 of crRNA-sp2 may be combined with nucleotides 4-89 of tracrRNA. Further options for obtaining an sgRNA can be derived from Nowak et al., 2016, Nucleic Acids Research, 44, 20, 9555-9564. For example, an sgRNA may be provided which comprises different forms of an upper stem structure, or in which the spacer sequence is differentially truncated from a canonical 20 nucleotides to 14 or 15 nucleotides. Further envisaged variants include those in which a putative RNAP III terminator sequence is removed from the lower stem. Also envisaged is a variant, in which the upper stem is extended to increase sgRNA stability and enhance its assembly with an sgRNA-guided nucleic acid-binding protein, e.g. Cas protein. According to further embodiments of the present invention, the sequence and form of the sgRNA may vary in accordance with the form or identity of the sgRNA-guided nucleic acid-binding protein, e.g. Cas protein. Accordingly, depending on the origin of said sgRNA-guided nucleic acid-binding protein, a different combination of sequence elements may be used. The present invention further envisages any future development in this context and includes any modification or improvement of the sgRNA-nucleic acid-binding protein interaction surpassing the information derivable from Jinke et al., 2012 or Nowak et al.,2016. In specific embodiments, the sgRNA to be used may have the sequence of any one of SEQ ID NO: 1 to 3. Particularly preferred is the use of an Streptococcus pyogenes sgRNA, e.g. as used in commercially available kits such as EnGen sgRNA synthesis Kit provided by New England Biolabs Inc. Also envisaged are similar sgRNA forms from other commercial suppliers, or individually prepared sgRNAs. Such sgRNAs may be derived from the sequence of SEQ ID NO: 1 if used with a cognate nucleic acid-binding protein form S. pyogenes. Alternatively, the sgRNA may be derived from the sequence of SEQ ID NO: 2 if used with a cognate nucleic acid-binding protein form Staphylococcus aureus. In a further alternative, the sgRNA may be derived from the sequence of SEQ ID NO: 3 if used with a cognate nucleic acid-binding protein form Streptococcus thermophilus.

The central principle of the present invention is the use of a sequence binding to a target DNA section within the sgRNA, wherein said binding sequence is specific for the WT version of the target DNA polynucleotide and is accordingly able to identify said sequence and distinguish it form other sequences, in particular mutant sequences differing from the binding section. In accordance with the CRSPR/Cas approach as defined above, the WT sequence which has been identified by the sgRNA can subsequently be cleaved by applying or adding a suitable sgRNA-guided nucleic acid-binding protein.

In preferred embodiments the "sgRNA-guided nucleic acid-binding protein" as used herein is a DNA binding Cas protein. Examples of such DNA binding Cas proteins are Cas2, Cas3, Cas5, Csn1 or Csx12 or Cas9. Also envisaged are derivatives thereof or mutants. In particularly preferred embodiments, the sgRNA-guided nucleic acid-binding protein is derived from a family of Cas9 proteins or derivatives thereof. It is even more preferred that the sgRNA-guided nucleic acid-binding protein is Cas9 or a derivative thereof. The derivative is preferably a functional derivative which has a nuclease activity. The present invention further envisages the use of Cas9 derived from different bacterial sources. For example, the Cas9 protein may be derived from Streptococcus pyogenes, Staphylococcus aureus, or Streptococcus thermophiles. It is preferred that the Cas9 is a Streptococcus pyogenes Cas9 protein. Further details on the form and use of Cas proteins may be derived from suitable literature sources such as Jiang and Doudna, 2017, Annu. Rev. Biophys., 46, 505-529, Makarova et al., 2011, Biology Direct, 6, 38 or Wang et al., 2016, Annu. Rev. Biochem., 85, 22.1-22.38.

The cleavage of WT sequences within the RCA concatemer via the sgRNA guided activity typically leads to the provision of several small fragments, for example corresponding in length to the original circular template of RCA due to the repetition of the sequence introduced the RCA method. Accordingly, a significant size difference between uncut (mutant) molecules and cleaved (WT) molecules is obtained. The term "uncut" molecule or polynucleotide as used herein relates to a target DNA polynucleotide which has not been recognized by a specific sgRNA as defined herein. Such polynucleotides may comprise any sequence difference with the sgRNA binding segment from the WT sequence. In certain embodiments, the sequence difference is a single nucleotide polymorphism. Also envisaged are insertions or deletions of 1, 2, 3, 4, 5, 6, 7, 8, 9, 10, 11, 12, 13, 14, 15, 16, 17, 18, 19, 20 or more nucleotides etc.

The size difference as mentioned above is exploited in the next step of the method, wherein uncut mutant target DNA polynucleotides are separated from cleaved WT fragments according to the size differences between these two DNA polynucleotide forms. This size selection step may be performed with any suitable method. For example, an agarose gel- or polyacrylamide gel-based approach or a bead based approach may be used. It is particularly preferred to use magnetic beads, which may bind under suitable conditions to DNA polynucleotides of different lengths.

Obtained target polynucleotides, i.e. DNA polynucleotides comprising a mutant sequence motif, may subsequently be purified, stored and/or used for additional activities.

In a final step of the method the uncut mutant target DNA polynucleotides are characterized. The term "characterization" as used herein relates to the determination of certain characteristics of the DNA polynucleotide. One of the characteristics to be determined according to the present invention is the length of the DNA polynucleotide. Another characteristic of the DNA polynucleotide to be determined according to the present invention is, in a further embodiment, the GC content of the DNA polynucleotide . Also envisaged is the identification of certain motifs or sequence stretches indicative for specific functions or their absence, or of the identity of the DNA polynucleotide. Particularly preferred is the characterization of the sequence of the DNA polynucleotide.

The term "characterization of the sequence" as used herein relates to any suitable sequencing methodology known to the skilled person. Preferably, a next-generation sequence (NGS) or second generation sequencing technique may be used, which is usually a massively parallel sequencing approach performed in a highly parallel fashion. The sequencing may, for example, be performed according to parallel sequencing approach on platforms such as Roche 454, GS FLX Titanium, Illumina, Life Technologies Ion Proton, Oxford Nanopore Technologies, Solexa, Solid or Helicos Biosciences Heliscope systems. The sequencing may, in certain embodiments, also include an additional preparation of polynucleotides, the sequencing, as well as subsequent imaging and initial data analysis steps.

Preparation steps for sequencing analyses may, for example, include cutting the polynucleotides with restriction enzymes which have cognate restriction enzyme recognition sites, preferably in the stem-loop oligonucleotide as described herein. Alternatively, the polynucleotides may be randomly broken into smaller sizes. Thereby sequencing templates such as fragment templates are generated. Accordingly, uncut concatemeric DNA polynucleotides may be size reduced to be compatible with a cognate sequencing method. Also envisaged is the direct sequencing of uncut concatemeric DNA polynucleotides with suitable sequencing techniques.

Spatially separated templates can, for example, be attached or immobilized at solid surfaces which allows for a sequencing reaction to be performed simultaneously. In typical examples, a library of nucleic acid fragments is generated and adaptors containing universal priming sites are ligated to the end of the fragments. Subsequently, the fragments are denatured into single strands and captured by beads. After amplification a huge number of templates may be attached or immobilized in a polyacrylamide gel, or be chemically crosslinked to an amino-coated glass surface, or be deposited on individual titer plates. Alternatively, solid phase amplification may be employed. In this approach forward and reverse primers are typically attached to a solid support. The surface density of amplified fragments is defined by the ratio of the primers to the template on the support. This method may produce millions of spatially separated template clusters which can be hybridized to universal sequencing primers for massively parallel sequencing reactions. Further suitable options include multiple displacement amplification methods. Suitable sequencing methods include, but are not limited to, cyclic reversible termination (CRT) or sequencing by synthesis (SBS) by Illumina, sequencing by ligation (SBL), single-molecule addition (pyrosequencing) or real-time sequencing. Exemplary platforms using CRT methods are Illumina/Solexa and HelicoScope. Exemplary SBL platforms include the Life/APG/SOLiD support oligonucleotide ligation detection. An exemplary pyrosequencing platform is Roche/454. Exemplary real-time sequencing platforms include the Pacific Biosciences platform and the Life/Visi-Gen platform. Other sequencing methods to obtain massively parallel nucleic acid sequence data include nanopore sequencing, sequencing by hybridization, nano-transistor array based sequencing, scanning tunneling microscopy (STM) based sequencing, or nanowire-molecule sensor based sequencing. Further details with respect to the sequencing approach would be known to the skilled person, or can be derived from suitable literature sources such as Goodwin et al., 2016, Nature Reviews Genetics, 17, 333-351, van Dijk et al., 2014, Trends in Genetics, 9, 418-426 or Feng et al., 2015, Genomics Proteomics Bioinformatics, 13, 4-16.

A size reduction of the uncut DNA polynucleotides may be obtained by shearing or fragmentation procedures in accordance with any suitable protocol known to the skilled person. Such methods include a restriction digest, adaptive focused acoustic shearing (AFA) or Covaris shearing, use of nebulization forces, sonication, point-sink shearing or the use of a French press shearing procedure. It is preferred to make use of a restriction enzyme digestion in a stem-loop oligonucleotide as described herein above. It is further preferred that the size of the polynucleotides obtained is similar or within a predefined range. Envisaged ranges are about 120 to about 400 nucleotides. Particularly preferred are sizes of about 150 to 300 nucleotides.

In particularly preferred embodiments, the characterization step (v) as mentioned above comprises additional sub-steps related to a transmembrane pore bases sequence characterization. Typically, such a characterization comprises the steps of: (v-a) ligating an adaptor polynucleotide associated with an DNA translocase enzyme and at least one cholesterol tether segment to the mutant target DNA polynucleotides obtained in step (iv); (v-b) contacting the modified DNA polynucleotide obtained in step (v-a) with a transmembrane pore such that the DNA translocase controls the movement of the DNA polynucleotide through the transmembrane pore and the cholesterol tether anchors the DNA polynucleotide in the vicinity of the transmembrane pore; and (v-c) taking one or more measurements during the movement of the DNA polynucleotide through said transmembrane pore, wherein the measurements are indicative of one or more characteristics of the DNA polynucleotide, thereby characterizing the target DNA polynucleotide.

The term "adaptor polynucleotide complex" as used herein refers to a complex of polynucleotides which comprises, inter alia, a sequence facilitating the entry of a DNA translocase enzyme into a transmembrane pore. In specific embodiments of the present invention said adaptor polynucleotide complex comprises a pair of two at least partially complementary polynucleotides. It is particularly preferred that said adaptor polynucleotide complex is attached to both strands of the DNA polynucleotide to allow for a characterization of both strands.

The portion of the adaptor complex which is associated with a DNA translocase enzyme may, in certain embodiments, comprise a leader sequence. Typically, said leader sequence threads into the transmembrane pore as described herein. The leader sequence may further comprise additional segments such as one or more spacers. The spacer may, for example, comprise a sequence which is capable of stalling the DNA translocase. It is particularly preferred that the leader sequence comprises a binding site for a DNA translocase enzyme. The term "DNA translocase enzyme binding site" as used herein includes a DNA or DNA analogue sequence of a length which allows one or more DNA translocase enzymes to bind thereto. The length of the binding site typically depends on the number of DNA translocase enzymes that should bind thereto. The region to which a DNA translocase enzyme is capable of binding is preferably a polynucleotide such as DNA, a modified polynucleotide (e.g. an abasic DNA), PNA, LNA, or polyethylene glycol (PEG). Preferably the DNA translocase enzyme binding site is a single stranded, non hybridized region. Accordingly, in preferred embodiments, said adaptor polynucleotide complex is pre-bound to one or more DNA translocases. The term "DNA translocase" as used herein relates to a motor protein, which is capable of interacting with a transmembrane pore as described herein and which accordingly transports a polynucleotide as single stranded entity through said pore, i.e. controls translocation of the a polynucleotide as described herein, e.g. DNA polynucleotide as defined above, preferably a concatemeric DNA polynucleotide as obtained in accordance with the present invention. Examples of suitable translocases include DNA helicases such as Hel308 helicase, RecD helicase, XPD helicase or Dda helicase.
In further embodiments, the leader sequence may comprise one or more blocking sites which are capable of preventing backwards movements of the DNA translocase enzyme or any slipping off said enzymes from the transmembrane pore.

The adaptor polynucleotide complex may further be associated to or comprise a tether segment. The term "tether segment" as used herein relates to an element which is capable of coupling the adaptor polynucleotide complex and any further element connected to it to a bilayer membrane. The coupling is typically transient and is conveyed by any suitable molecule, preferably a cholesterol entity or a fatty acid, more preferably a cholesterol entity such as a cholesterol-TEG molecule. The coupling accordingly helps to anchor the adaptor polynucleotide complex and its associated elements at or close to the transmembrane pore and thereby allows for an introduction of the DNA polynucleotide of the opposite stand to enter the transmembrane pore and to be characterized. It is particularly preferred that said tether segment is provided on both strands of the DNA polynucleotide to allow for a characterization of both strands.

Alternative compounds which can be used to couple to a membrane comprise biotin, thiol or lipids. The tether typically comprises, besides the coupling functionality, a non-RNA polynucleotide, which is connected to said coupling entity, e.g. a cholesterol entity. The tether segment may further comprise one or more linker segments, e.g. a portion of variable length, which can be employed to increase the distance between the target DNA polynucleotide and the transmembrane pore to facilitate its characterization. The linker may, in further embodiments, comprise a DNA translocase enzyme binding site as defined herein above.

The connection of the polynucleotide complex to the polynucleotide obtained in the previous step may be performed by ligating steps. Alternatively, any other suitable connection approach may be used, e.g. chemical attachment via click chemistry or covalent bondings etc. It is preferred that said connection is performed such that the DNA translocase enzyme is connected to the DNA polynucleotide to be characterized, and that the tether element is connected to the complementary strand.

In a further step the modified DNA polynucleotide obtained in the previous step (v-a) is contacted with a transmembrane pore such that the DNA translocase controls the movement of the DNA polynucleotide through the transmembrane pore and the cholesterol tether anchors the DNA polynucleotide in the vicinity of the transmembrane pore. Typically, the function of a tether anchor as described herein is to bring the molecules to the membrane surface, where the transmembrane pore is located. In this scenario, the characterization of the DNA polynucleotide is facilitated since the transmembrane pore can be reached more easily. The term "transmembrane pore" as used herein relates to a protein spanning a bilayer membrane which comprises an opening which is capable of guiding through a polynucleotide. The transmembrane pore may be any suitable protein. Examples of preferred transmembrane proteins include a protein pore derived from hemolysin, leukocidin, MspA, MspB, MspC, MspD, CsgG, lysenin, outer membrane porin F (OmpF), outer membrane porin G (OmpG), outer membrane phospholipase A, Neisseria autotransporter lipoprotein (NalP) or WZA. Also envisaged are commercially available transmembrane pore proteins such as the pore proteins offered by, or described by Oxford Nanopore Technology.

In an ultimate step (v-c) one or more measurements are taken during the movement of the DNA polynucleotide through said transmembrane pore. Said measurements may be indicative of one or more characteristics of the DNA polynucleotide, which allows to characterize the target DNA polynucleotide as defined herein above, in particular the sequence of the DNA polynucleotide. The term "measurement" as used herein relates to optical and/or electrical measurements, preferably to electrical measurement at the transmembrane pore. Typically, the current passing through the transmembrane pore is measured as the target DNA polynucleotide passes through the transmembrane pore. The measured current is typically indicative for one or more characteristics of the analyzed polynucleotides. The method may, for example, be performed using an apparatus as described in the prior art, e.g. disclosed in principle in WO 2008/102120, or derivatives or modified versions thereof. In general, the methods may be carried out using a patch claim or voltage clamp to detect changes in the current across the transmembrane pore when the polynucleotide is translocated through said pore. The measurement, in certain embodiments, includes the use of a charge carrier such as metal salts, chloride salts, ionic liquids, organic salts, in particular NaCl, KCl, CsCl; further envisaged is the use of a suitable buffer, e.g. HEPES, Tris-HCl etc.; further envisaged is the use of nucleotides, e.g. AMP, ADP, ATP, dAMP, dADP, dATP etc. which may be employed for the translocase activity; and enzyme cofactors such as divalent metals ions including Mg2+, Ca²⁺, Co²⁺.

In a further aspect the present invention relates to a kit comprising one or more oligonucleotides specific for at least a portion of the target DNA polynucleotide, preferably as defined herein above, a synthetic single guide RNA (sgRNA) specific for the WT version of the target DNA polynucleotide, preferably as defined herein above, and an sgRNA-guided nucleic acid-binding protein, preferably as defined herein above. The kit is preferably for characterizing a target DNA polynucleotide. The features of the methods as defined herein above apply also to the kit of the present invention. The kit may, for example, comprise reagents and components as defined in one or more steps of the present methods, or being known to the skilled person. For example, the kit may comprise reagents or components for performing RCA on the basis of one or more oligonucleotides as defined herein. It may, in addition, comprise reagents and components for subsequently repairing the RCA products such as a T7 endonuclease and/or a DNA polymerase and optionally also a ligase as described herein. The kit may, alternatively or additionally, comprise reagents and components for cleaving a WT version of the target DNA polynucleotide with an sgRNA-guided nucleic acid-binding protein as defined herein. In a different embodiment, the kit may comprise or may comprise in addition reagents or components for performing a size selection. The kit may, in general, comprise suitable buffer solutions, labels or washing liquids etc. Furthermore, the kit may comprise an amount of a known nucleic acid molecule or protein, which can be used for a calibration of the kit or as an internal control. Corresponding ingredients would be known to the skilled person.

In a further preferred embodiment, the kit may comprise or comprise in addition, components necessary for the performance of sequencing reactions. It is, in particular, preferred to provide within the kit components and reagents require for transmembrane pore sequencing approaches. For example, the kit may comprise or may comprise in addition reagents or components for connecting an adaptor polynucleotide complex associated with a DNA translocase enzyme and at least one cholesterol tether segment to the polynucleotide as described herein. In a further embodiment, the kit may comprise or may comprise in addition reagents or components for contacting the target DNA polynucleotide as defined herein with a transmembrane pore such that the DNA translocase controls the movement of the target DNA polynucleotide through the transmembrane pore and the cholesterol tether anchors the target DNA polynucleotide in the vicinity of the transmembrane pore. In yet another embodiment, the kit may comprise or may comprise in addition reagents or components taking one or more measurements during the movement of the target DNA polynucleotide through the transmembrane pore, wherein the measurements are indicative of one or more characteristics of the target DNA polynucleotide, thereby characterizing the target DNA polynucleotide, as defined above. The kit may further comprise two ore more of the component or reagent groups as defined above, e.g. components or reagents for performing 2 steps as defined herein, 3 steps as defined herein, 4 steps as defined herein etc.

Additionally, the kit may comprise an instruction leaflet and/or may provide information as to its usage etc.

Also envisaged is an apparatus performing the above mentioned method steps. The apparatus may, for example, be composed of different modules which can perform one or more steps of the method of the present invention. These modules may be combined in any suitable fashion, e.g. they may be present in a single place or be separated. Also envisaged is the performance of the method at different points in time and/or in different location. Some steps of the method as define herein may be followed by breaks or pauses, wherein the reagents or products etc. are suitably stored, e.g. in a freezer or a cooling device. In case these steps are performed in specific modules of an apparatus as defined herein, said modules may be used as storage vehicle. The modules may further be used to transport reaction products or reagents to a different location, e.g. a different laboratory etc.

Also envisaged by the present invention is the use of one or more of the kit components as described above for the characterization of a target DNA polynucleotide.

Turning now to FIGURE 1, a schematic illustration of the steps for characterizing a target DNA polynucleotide using rolling circle amplification (RCA) and a synthetic single guide RNA (sgRNA) according to an embodiment of the present invention is shown. In a first step a DNA polynucleotide 1 representing a mixture of target DNA polynucleotides is provided. The DNA polynucleotide is modified 2 by end-repairing and T-tailing 3 activities. Subsequently, a stem-loop oligonucleotide 4 with a compatible 3' T overhang is connected 5 to the DNA polynucleotide. This step yields a ds DNA polynucleotide 6 with both termini comprising the stem-loop oligonucleotide 4. Subsequently an oligonucleotide 8 specific for at least a portion of the target DNA polynucleotide is annealed 7 to the modified DNA polynucleotide 6. The next step is a rolling circle amplification (RCA) 9 which is followed by the provision 10 of ds DNA polynucleotides via the activity of a DNA polymerase and optionally a ligase. This step yields a mixture of concatemeric DNA polynucleotides either representing a WT sequence 12 or a mutant sequence 13. The concatemers are processed 14 with an sgRNA and an sgRNA-guided nucleic acid binding protein such as Cas 9 into smaller fragments 15 in case the DNA polynucleotides represent WT sequences 12. In case the DNA polynucleotides represent mutant sequences they remain uncut 13. The uncut mutant DNA polynucleotides are subsequently separated from the WT sequences via size selection 16. They can further be modified and used for a transmembrane pore 17 based sequencing approach 18, which is performed in a suitable sequencing device 19.

### LIST OF REFERENCE NUMERALS

- 1: DNA polynucleotide representing a mixture of target DNA polynucleotides
- 2: Modification of DNA polynucleotide
- 3: A-tailed DNA polynucleotide
- 4: Stem-loop oligonucleotide
- 5: Connection of stem-loop oligonucleotide and DNA polynucleotide
- 6: ds DNA polynucleotide with stem-loop oligonucleotides at both termini
- 7: Annealing reaction
- 8: Oligonucleotide specific for at least a portion of the target DNA polynucleotide
- 9: Rolling circle amplification (RCA)
- 10: Provision of ds DNA polynucleotides
- 11: Mixture of concatemeric DNA polynucleotides
- 12: Concatemer of WT sequences
- 13: Concatemer of mutant sequences
- 14: Processing with an sgRNA and an sgRNA-guided nucleic acid binding protein
- 15: Fragment of concatemer of WT sequences
- 16: Size selection of mutant concatemers
- 17: Transmembrane pore
- 18: Sequencing reaction
- 19: Sequencing device

The following figure is provided for illustrative purposes. It is thus understood that the figure is not to be construed as limiting. The skilled person in the art will clearly be able to envisage further modifications of the principles laid out herein.

## Claims

1. A method of characterizing a target DNA polynucleotide comprising:
(i) providing a mixture of DNA polynucleotides comprising at least a wildtype (WT) version and a mutant version of said DNA polynucleotide;
(ii) providing a pool of amplified and concatenated DNA polynucleotides by amplifying said mixture of DNA polynucleotides of step (i) by rolling circle amplification (RCA);
(iii) identifying and cleaving the WT version of the target DNA polynucleotide by using a synthetic single guide RNA (sgRNA) specific for said WT version and an sgRNA-guided nucleic acid-binding protein, preferably Cas9;
(iv) size selecting uncut mutant target DNA polynucleotides; and
(v) characterizing the uncut mutant target DNA polynucleotides.

2. The method of claim 1, wherein said step (v) comprises the following sub-steps:
(v-a) ligating an adaptor polynucleotide associated with an DNA translocase enzyme and at least one cholesterol tether segment to the mutant target DNA polynucleotides obtained in step (iv) ;
(v-b) contacting the modified DNA polynucleotide obtained in step (v-a) with a transmembrane pore such that the DNA translocase controls the movement of the DNA polynucleotide through the transmembrane pore and the cholesterol tether anchors the DNA polynucleotide in the vicinity of the transmembrane pore; and
(v-c) taking one or more measurements during the movement of the DNA polynucleotide through said transmembrane pore, wherein the measurements are indicative of one or more characteristics of the DNA polynucleotide, thereby characterizing the target DNA polynucleotide.

3. The method of claim 1 or 2, additionally comprising after step (i) a step (i-a) of end-repairing and A-tailing of the DNA polynucleotide.

4. The method of claim 3, additionally comprising after step (i-a) a step (i-b) of circularizing the DNA polynucleotide with a stem-loop oligonucleotide, wherein said stem-loop oligonucleotide comprises a barcoding sequence and a restriction enzyme recognition site.

5. The method of any one of claims 1 to 4, wherein the rolling circle amplification is performed with one or more oligonucleotides specific for at least a portion of the target DNA polynucleotide.

6. The method of claim 5, wherein said one or more oligonucleotides specific for at least a portion of the target DNA polynucleotide are hexamers, heptamers, and/or octamers.

7. The method of any one of claims 1 to 6, wherein said rolling circle amplification is performed until the amplified DNA polynucleotide has a size of at least about 300 nucleotides, preferably a size of about at least 3000 nucleotides.

8. The method of any one of claims 5 to 7, wherein the rolling circle amplification products obtained are repaired using a T7 endonuclease, DNA polymerase and optionally a ligase.

9. The method of any one of claims 1 to 8, wherein said target DNA polynucleotide represents a gene, one or more exons of a gene, an intergenic region, a non-transcribed regulatory region, and/or an open reading frame or a sub-portion thereof; or a panel of different genes, a panel of one or more exons of different genes, a panel of intergenic regions, a panel of non-transcribed regulatory regions, and/or a panel of open reading frames or sub-portions thereof, or any combination of any of the before mentioned elements.

10. The method of any one of claims 1 to 9, wherein said target DNA polynucleotide is cell free DNA (cfDNA), preferably derived from a liquid biopsy.

11. The method of any one of claims 1 to 10, wherein said characterization of the DNA polynucleotide is (i) a determination of the length of the DNA polynucleotide, (ii) a determination of the identity of the DNA polynucleotide, or (iii) a determination of the sequence of the DNA polynucleotide; preferably the sequence of the DNA polynucleotide.

12. The method of any one of claims 2 to 11, wherein said DNA translocase is a DNA helicase such as Hel308 helicase, RecD helicase, XPD helicase or Dda helicase.

13. The method of any one of claims 2 to 12, wherein said transmembrane pore is a protein pore derived from hemolysin, leukocidin, MspA, MspB, MspC, MspD, CsgG, lysenin, outer membrane porin F (OmpF), outer membrane porin G (OmpG), outer membrane phospholipase A, Neisseria autotransporter lipoprotein (NalP) or WZA.

14. A kit for characterizing a target DNA polynucleotide comprising one or more oligonucleotides specific for at least a portion of the target DNA polynucleotide, a synthetic single guide RNA (sgRNA) specific for the WT version of the target DNA polynucleotide and an sgRNA-guided nucleic acid-binding protein, preferably a Cas9 endonuclease.

15. The kit of claim 14, additionally comprising a DNA translocase and a cholesterol tether.
